# EUROPÄISCHE PATENTSCHRIFT

(11) **EP 4 521 351 B1**
(45) Veröffentlichungstag und Bekanntmachung des Hinweises auf die Patenterteilung: **18.02.2026**
(21) Anmeldenummer: 23196632.6
(22) Anmeldetag: 11.09.2023
(51) Int. Cl.: G06T 11/00

(54) **VERFAHREN UND VORRICHTUNG VERFAHREN ZUM ERZEUGEN EINES ERGEBNISBILDES BASIEREND AUF EINEM TOMOSYNTHESE-BILDDATENSATZ EINER BRUST**
METHOD AND DEVICE FOR GENERATING A RESULT IMAGE BASED ON A TOMOSYNTHESIS IMAGE DATA SET OF A BREAST
PROCÉDÉ ET APPAREIL DE GÉNÉRATION D'IMAGE DE RÉSULTAT SUR LA BASE D'UN ENSEMBLE DE DONNÉES D'IMAGE DE TOMOSYNTHÈSE DU SEIN

(43) Veröffentlichungstag der Anmeldung: 12.03.2025
(73) Patentinhaber: Siemens Healthineers AG, 91301 Forchheim (DE)
(72) Erfinder: Ritschl, Ludwig, 96155 Buttenheim (DE)
(74) Vertreter: Siemens Healthineers Patent Attorneys

(56) Entgegenhaltungen:
- US-A1- 2021 113 169
- SECHOPOULOS IOANNIS: "Breast Imaging : Diagnosis and Intervention", November 2022 (2022-11-01), Cham, pages 1 - 24, XP093132932, ISSN: 0942-5373, ISBN: 978-3-030-94918-1, Retrieved from the Internet <URL:https://link.springer.com/content/pdf/10.1007/978-3-030-94918-1_1> DOI: 10.1007/978-3-030-94918-1_1

## Beschreibung

Die Erfindung betrifft ein Verfahren und eine Vorrichtung zum Erzeugen eines Ergebnisbildes basierend auf einem Tomosynthese-Bilddatensatz einer Brust sowie ein entsprechendes Mammographiesystem. Die Erfindung befasst sich insbesondere mit einer nicht-planaren Nachbearbeitung von spektralen Röntgenbildern.

Bei der Untersuchung der weiblichen Brust stellt die Tomosynthese eine vorteilhafte Technik zur Erstellung von dreidimensionalen Bildern dar. Typischerweise wird bei der digitalen Brusttomosynthese ein kubisches Volumen über dem Detektor rekonstruiert, in dem die Brust liegt. Die Höhe des Volumens wird typischerweise durch die Kompressionsdicke der Brust definiert.

Das kubische Volumen enthält planare Schnitte durch die Brust. Die Rekonstruktion kann dabei so erfolgen, dass die planaren Schnitte innerhalb des Volumens in ein perspektivisches Koordinatensystem rekonstruiert werden. Dieses perspektivische Koordinatensystem hilft dabei, entlang der räumlich variierenden Richtung in Z-Auflösung abzutasten, was ein wesentliches Merkmal der DBT-Rekonstruktion ist. Bei dieser Abtastung kann jeder Strahl typischerweise mit zwei Winkeln α und φ relativ zum Normalenvektor des Detektors (Z-Achse) parametrisiert werden.

Im Falle eines gebogenen Kompressionspaddels oder einer alternativen Kompressions- bzw. Fixierungsmethode, die die natürliche gekrümmte Form der Brust bewahrt, führt diese Rekonstruktion mit planaren Schnitten zu einer nicht optimalen Darstellung von DBT-Schnitten, die nur kleinere Teile der Brust in den außenliegenden Schnitten zeigen.

Es ist eine Aufgabe der vorliegenden Erfindung, ein verbessertes Verfahren und eine verbesserte Vorrichtung zum Erzeugen eines Ergebnisbildes basierend auf einem Tomosynthese-Bilddatensatz einer Brust anzugeben, mit dem die oben beschriebenen Nachteile vermieden werden und die insbesondere eine nicht-planare Nachbearbeitung von spektralen Röntgenbildern ermöglichen.

Diese Aufgabe wird durch ein Verfahren gemäß Patentanspruch 1, eine Vorrichtung gemäß Patentanspruch 8, sowie durch ein Mammographiesystem gemäß Patentanspruch 9 gelöst.

Das erfindungsgemäße Verfahren dient zum Erzeugen eines Ergebnisbildes basierend auf einem Tomosynthese-Bilddatensatz einer Brust, welche mit einer Kompressionsplatte auf einem Detektor fixiert worden ist. Dabei weist eine der Brust zugewandte Berührungsoberfläche von Kompressionsplatte und/oder Detektor eine konkave Wölbung auf. Die Brust wird also nicht zwischen zwei ebenen Platten gehalten und damit oben und unten zu einer planaren Fläche gedrückt, sondern weist oben und/oder unten eine Wölbung auf. Da die Platten konkav gewölbt sind, ist die dazwischenliegende Brust konvex gewölbt.

Das Verfahren umfasst die folgenden Schritte:
- Definieren von gewölbten Schichten zwischen Kompressionsplatte und Detektor, welche in ihrer Form der Wölbung von Kompressionsplatte und/oder Detektor folgen oder einen Übergang zwischen diesen Wölbungen darstellen,
- Rekonstruieren von (gewölbten) Schichtbildern, welche auf den gewölbten Schichten liegen,
- Erzeugen des Ergebnisbildes als Schichtstapel der (gewölbten) Schichtbilder,
- Ausgeben des Ergebnisbildes.

Die Brust wird also bei einer Aufnahme von der Kompressionsplatte auf dem Detektor gehalten. Zumindest eine dieser beiden Oberflächen, welche die Brust komprimieren, ist konkav gewölbt. Dies bedeutet, dass diese Oberfläche eine konvexe Wölbung der Brust erlaubt. Eine konkave Wölbung kann z.B. dadurch erreicht werden, dass die Kompressionsplatte an ihren Rändern nach unten gebogen ist, wie eine Schale oder dass sie eine ebene Platte mit einer konvexen Ausnehmung an ihrer Unterseite darstellt. Die Oberfläche über dem Detektor kann aber auch eine Kuhle aufweisen. Die Oberseite und/oder die Unterseite der aufgenommenen Brust ist also konvex gewölbt.

Es werden nun entsprechend der Wölbung der Brust, welche in ihrer Form der Wölbung von Kompressionsplatte und/oder Detektor entspricht, gewölbte Schichten zwischen Kompressionsplatte und Detektor definiert. Diese folgen der Wölbung der Brust oder stellen einen Übergang zwischen unterschiedlichen Wölbungen dar. Es ist bevorzugt, dass die äußersten Schichten im Wesentlichen der Wölbung der Brust folgen, wobei "im Wesentlichen" zu mindestens 80%, insbesondere zu mindestens 90%, entspricht. Die dazwischenliegenden Schichten sollten einen (insbesondere regelmäßigen) Übergang zwischen den Wölbungen der äußersten Schichten darstellen.

Hier können im Grunde zwei Fälle unterschieden werden: Bei dem einen Fall ist die Brust an einer Seite gewölbt und an der anderen Seite eben (wobei eine ebene Schicht an der ebenen Seite dort der "Wölbung" der Brust entspricht), bei dem anderen Fall ist die Brust oben und unten gewölbt und die obere Wölbung baucht nach oben und die untere Wölbung baucht nach unten.

Die dazwischenliegenden Schichten sollten dann einen Übergang von Wölbung zu einer Ebene bilden bzw. zwischen einer Wölbung nach oben und einer Wölbung nach unten (ggf. mit einer ebenen Schicht in der Mitte).

Eine Bildrekonstruktion ist im Grunde im Stand der Technik bekannt. Bei dem hier geschilderten Verfahren werden nun jedoch keine ebenen (planaren) Schichtbilder rekonstruiert, sondern Schichtbilder auf den gewölbten Schichten (wobei insbesondere eine dieser Schichten auch eben sein kann, z.B. die mittlere Schicht einer bikonvexen Brust oder die äußerste Schicht einer monokonvexen Brust. Es ergeben sich als im Grunde gewölbte Schichtbilder.

Hier sollte beachtet werden, dass die Schichten die theoretischen Ebenen sind, mittels denen die Rekonstruktion durchgeführt wird und die Schichtbilder die letztlich rekonstruierten Bilder.

Aus diesen Schichtbildern wird dann das Ergebnisbild als Schichtstapel erzeugt und ausgegeben, z.B. werden die Schichtbilder einfach übereinander gelegt.

Es werden also direkt (gewölbte) Schichtbilder in dem aufgenommenen Volumen rekonstruiert. Die Wölbung kann auf der bekannten Wölbung von Kompressionsplatte bzw. der Oberfläche über dem Detektor basieren. Diese direkte Rekonstruktion hat den Vorteil, dass sich keine zu kurzen (planaren) Schichten ergeben.

Eine erfindungsgemäße Vorrichtung dient zum Erzeugen eines Ergebnisbildes basierend auf einem Tomosynthese-Bilddatensatz einer Brust, welche mit einer Kompressionsplatte auf einem Detektor fixiert worden ist, wobei die der Brust zugewandte Berührungsoberfläche von Kompressionsplatte und/oder Detektor eine konkave Wölbung aufweist. Die Vorrichtung ist dabei bevorzugt zur Ausführung des erfindungsgemäßen Verfahrens ausgelegt und umfasst die folgenden Komponenten:
- eine Schichteinheit ausgelegt zum Definieren von gewölbten Schichten zwischen Kompressionsplatte und Detektor, welche in ihrer Form der Wölbung von Kompressionsplatte und/oder Detektor folgen oder einen Übergang zwischen diesen Wölbungen darstellen,
- eine Rekonstruktionseinheit, ausgelegt zum Rekonstruieren von (gewölbten) Schichtbildern, welche auf den gewölbten Schichten liegen
- eine Ergebniseinheit, ausgelegt zum Erzeugen des Ergebnisbildes als Schichtstapel der (gewölbten) Schichtbilder,
- eine Datenschnittstelle ausgelegt zum Ausgeben des Ergebnisbildes.

Die Funktion der Einheiten wurde oben bereits im Zuge des Verfahrens beschrieben.

Ein erfindungsgemäßes Mammographiesystem umfasst eine erfindungsgemäße Vorrichtung und/oder ist zum Durchführen eines erfindungsgemäßen Verfahrens ausgelegt.

Die Erfindung kann insbesondere in Form einer Rechnereinheit, insbesondere in einer Steuereinrichtung für ein Mammographiesystem, mit geeigneter Software realisiert sein. Die Rechnereinheit kann z.B. hierzu einen oder mehrere zusammenarbeitende Mikroprozessoren oder dergleichen aufweisen. Insbesondere kann sie in Form von geeigneten Softwareprogrammteilen in der Rechnereinheit realisiert sein. Eine weitgehend softwaremäßige Realisierung hat den Vorteil, dass auch schon bisher verwendete Rechnereinheiten auf einfache Weise durch ein Software- bzw. Firmware-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Rechnereinheit ladbar ist, mit Programmabschnitten, um alle Schritte des erfindungsgemäßen Verfahrens auszuführen, wenn das Programm in der Rechnereinheit ausgeführt wird. Ein solches Computerprogrammprodukt kann neben dem Computerprogramm gegebenenfalls zusätzliche Bestandteile wie z.B. eine Dokumentation und/oder zusätzliche Komponenten, auch Hardware-Komponenten, wie z. B. Hardware-Schlüssel (Dongles etc.) zur Nutzung der Software, umfassen. Zum Transport zur Rechnereinheit und/oder zur Speicherung an oder in der Rechnereinheit kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Gemäß einem bevorzugten Verfahren entspricht die Wölbung einer ersten Schicht, welche benachbart zur Kompressionslatte ist, im Wesentlichen der Form der Berührungsoberfläche der Kompressionsplatte. Wie oben gesagt, meint hier "im Wesentlichen", dass die Wölbung bevorzugt zu 80%, insbesondere zu 90% der Wölbung der Kompressionsplatte entspricht (z.B. dem Wölbungsradius). Ihre Wölbung kann identisch zu der Wölbung der Kompressionsplatte sein, jedoch ist in dem Fall, dass die erste Schicht etwas von der Kompressionsplatte entfernt ist, bevorzugt, dass die Wölbung der Schicht bereits ein wenig an die gegenüberliegende Wölbung angepasst ist.

Gemäß einem bevorzugten Verfahren entspricht die Wölbung einer zweiten Schicht, welche benachbart zum Detektor ist, im Wesentlichen der Form der Berührungsoberfläche des Detektors. Ebenfalls meint hier "im Wesentlichen", dass die Wölbung bevorzugt zu 80%, insbesondere zu 90% der Wölbung der entsprechenden Oberfläche entspricht (z.B. dem Wölbungsradius). Ihre Wölbung kann identisch zu der Wölbung der Oberfläche sein, jedoch ist in dem Fall, dass die zweite Schicht etwas von der Oberfläche entfernt ist, bevorzugt, dass die Wölbung der Schicht bereits ein wenig an die gegenüberliegende Wölbung angepasst ist.

Gemäß einem bevorzugten Verfahren folgt die Wölbung einer der dazwischenliegenden Schichten einem regelmäßigen Übergang zwischen der Wölbung der ersten Schicht und der zweiten Schicht. Ein solcher regelmäßiger Übergang ist z.B. ein regelmäßiger Übergang der Krümmungsradien.

Bevorzugt ist die Berührungsoberfläche des Detektors eben und die zweite Schicht ebenfalls eben. Die Wölbung der weiteren Schichten nimmt dann bevorzugt von der zweiten Schicht ausgehend bis zur ersten Schicht stetig zu.

Gemäß einem bevorzugten Verfahren wird die Wölbung der ersten Schicht und/oder der zweiten Schicht basierend auf einer Vermessung der jeweils benachbarten Berührungsoberfläche bestimmt. Da sowohl die Oberfläche der Kompressionsplatte als auch die Oberfläche über dem Detektor bekannt ist (bzw. gut vermessen werden kann), ist dies einfach durchzuführen. Es sollte beachtet werden, dass die komprimierte Brust diesen Formen folgt und damit auch die Form der Brust bekannt ist bzw. einfach bestimmt werden kann.

Gemäß einem bevorzugten Verfahren wird die Wölbung der ersten Schicht und/oder der zweiten Schicht basierend auf Daten eines Sensors bestimmt, welcher die Form der Oberfläche der Brust misst. Diese Vorgehensweise hat den Vorteil, dass Anpassungsfehler der Brust an die sie komprimierenden Oberflächen vermieden werden.

Gemäß einem bevorzugten Verfahren wird für die gewölbten Schichten ein Koordinatensystem (xₙ, yₙ, zₙ) konstruiert, bei dem die (xₙ, yₙ) bei gleichem n jeweils in einer Schicht liegen und die zₙ über die Fläche (xₙ, yₙ) die Z-Position der Punkte der gewölbten Schicht vorgeben (also die Höhe über dem Detektor). Dieses Koordinatensystem gibt damit direkt die gewölbten Schichten vor.

Konkret kann man die Koordinaten (xₙ, yₙ, zₙ) mittels einer Funktion F in einem kartesischen Koordinatensystem berechnen.

In einem einfachen Beispiel könnten (xₙ, yₙ) einer gewölbten Schicht den (x, y) einer ebenen Schicht eines kartesischen Koordinatensystems entsprechen, also (xₙ, yₙ) = (x, y). Gegenüber dieser ebenen Schicht könnten die zₙ der gewölbten Schicht sich aus einer vorgegeben Funktion F berechnen, welche die Wölbung definiert. Dabei können die Schichten z.B. wie folgt berechnet werden:
- zₙ = Fₙ(x, y) für individuelle Funktionen Fₙ für die einzelnen Schichten,
- zₙ = F(x, y, n) für eine gemeinsame, von n abhängige Funktion F für alle Schichten,
- zₙ = F(x, y, z) für eine von z abhängige Funktionen F für alle Schichten für ausgewählte Werte z, z.B. die z der ebenen Schichten.

Gemäß einem bevorzugten Verfahren basiert die Rekonstruktion von Schichtbildern auf einer Berechnung der Wege von Röntgenstrahlen, die durch die gewölbten Schichten dringen. Für eine bevorzugte Projektion der Schichten werden Koordinatenpaare zwischen einem Koordinatensystem (xₙ, yₙ, zₙ) und einem Koordinatensystem (x, y, z) bestimmt, in dem eine Vielzahl von Strahlen mit den Winkeln (α, φ) zwischen ihren Schnittpunkten mit dem Detektor und der Objektoberfläche in äquidistanten Schritten abgetastet werden.

Hierzu sollte man sich der Anschaulichkeit halber vorstellen, dass bei einer Rekonstruktion von Schichtbildern aus einem Tomosynthese-Bilddatensatz (der z.B. aus einer Vielzahl von Projektionsbildern besteht) jeweils von einem Voxel ausgegangen wird, von dem aus dem Tomosynthese-Bilddatensatz unter Verwendung bekannter Rechenoperationen (im kartesischen Koordinatensystem) der Voxelwert berechnet wird. Es kann nun die Position eines jeden Punktes (xₙ, yₙ, zₙ) der gewölbten Schichten in diesem kartesischen Koordinatensystem bestimmt werden. Also kann für jedes Voxel an der Position (xₙ, yₙ, zₙ) auch der Voxelwert berechnet werden. Man erhält dadurch alle benötigten Voxelwerte für die gewölbten Schichten.

Es sollte beachtet werden, dass der Röntgenstrahl bei der Aufnahme in der Regel kegelförmig ist. Dies sollte bei der Rekonstruktion berücksichtigt werden. Die Kegelform führt zum einen dazu, dass bei jedem Projektionsbild des Tomosynthese-Bilddatensatz eine Vielzahl von Strahlen mit unterschiedlichen Winkeln (α, φ) emittiert wird, als auch, dass die Strahlintensität in einem Voxel einer oberen Schicht größer ist als die eines Voxels einer unteren Schicht. Dies lässt sich jedoch mit Korrekturrechnungen kompensieren. Diese Korrekturrechnungen sind in einem kartesischen Koordinatensystem bekannt. Hier kann nun ebenfalls die Position eines jeden Punktes (xₙ, yₙ, zₙ) der gewölbten Schichten in diesem kartesischen Koordinatensystem bestimmt werden. Also kann für jedes Voxel an der Position (xₙ, yₙ, zₙ) auch die Korrekturrechnung vorgenommen werden. Man erhält dadurch alle benötigten Kompensationen für die gewölbten Schichten. Gemäß einem bevorzugten Verfahren berücksichtigt die Rekonstruktion die Kegelform eines aufnehmenden Röntgenstrahls. Dabei wird die Position einer Vielzahl von Punkten (xₙ, yₙ, zₙ) der gewölbten Schichten (in einem kartesischen Koordinatensystem) bestimmt und eine Korrekturrechnung zur Kompensation der Kegelform des Röntgenstrahls für diese Punkte vorgenommen.

Innerhalb der Rückprojektionsschleife kann dann basierend auf den Koordinatenpaaren eine spezielle Berechnung oder Suche durchgeführt werden (die je nach Leistung optimiert werden sollte). Diese Berechnung führt einen Punkt im Koordinatensystem (xₙ, yₙ, zₙ) in einen Punkt im Koordinatensystem (x, y, z) über. In einem kartesischen Koordinatensystem kann dies bevorzugt über eine einfache lineare Transformation erfolgen.

Gemäß einem bevorzugten Verfahren wird also als Ergebnisbild ein dreidimensionales Bild der Brust in kartesischen Koordinaten erstellt. Bevorzugt werden dabei Bildkoordinaten der Schichtbilder mittels einer linearen Transformation in kartesische Koordinaten überführt.

Gemäß einem bevorzugten Verfahren werden die Schichten orthogonal zur Flächennormalen der Berührungsoberfläche des Detektors ausgerichtet. Die Schichtbilder bilden dann in Richtung dieser Flächennormalen aufeinander geschichtet den Schichtstapel. Dies ermöglicht eine sehr einfache Konstruktion des Ergebnisbildes.

Die Erfindung beschreibt ein vorteilhaftes Sampling-Verfahren, welche die Form der Brust berücksichtigt und maximalen Nutzen aus dem Inhalt der Schichtbilder ziehen lässt. Vorteilhaft ist auch, dass die nicht-planaren Sampling-Geometrien direkt zur Rekonstruktion verwendet werden können. Dies vermeidet zusätzliche Interpolationsschritte, die z.B. die Sichtbarkeit von Mikro-Kalzifikationen verschlechtern könnten.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine grob schematische Darstellung eines bevorzugten Mammographiesystems mit einer bevorzugten Vorrichtung,
Figur 2 einen Ablaufplan für einen möglichen Ablauf eines erfindungsgemäßen Verfahrens,
Figur 3 ein Beispiel für eine komprimierte Brust mit einer Rekonstruktion mit ebenen Schichten gemäß dem Stand der Technik,
Figur 4 ein Beispiel für eine gewölbt komprimierte Brust mit einer Rekonstruktion mit ebenen Schichten gemäß dem Stand der Technik,
Figur 5 ein Beispiel für eine gewölbt komprimierte Brust mit einer Rekonstruktion mit gewölbten Schichten gemäß dem erfindungsgemäßen Verfahren,
Figur 6 ein Beispiel für eine gewölbt komprimierte Brust mit einer Rekonstruktion mit gewölbten Schichten gemäß dem erfindungsgemäßen Verfahren.

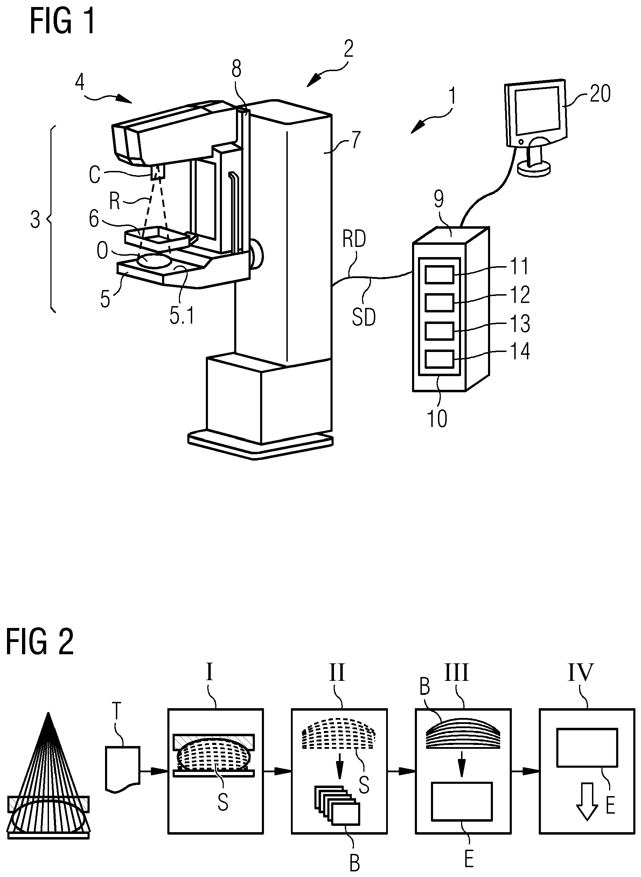

In Figur 1 ist beispielhaft und grob schematisch ein Mammographiesystem 1 in Form eines Tomosynthesesystems 1 gezeigt. Relative Richtungsangaben wie "oben", "unten" etc. beziehen sich auf ein bestimmungsgemäß für den Betrieb aufgestelltes Tomosynthesesystem 1. Das Tomosynthesesystem 1 umfasst ein Tomosynthesegerät 2 und eine Steuereinrichtung 9.

Das Tomosynthesegerät 2 weist eine Standsäule 7 und eine Quelle-Detektor-Anordnung 3 auf, die wiederum eine Röntgenstrahler 4 und einen Detektor 5 mit einer Detektorfläche 5.1 umfassen. Die Standsäule 7 steht im Betrieb auf dem Untergrund. Mit ihr ist die Quelle-Detektor-Anordnung 3 verschiebbar verbunden, sodass die Höhe der Detektorfläche 5.1, also der Abstand zum Untergrund, auf eine Brusthöhe einer Patientin eingestellt werden kann.

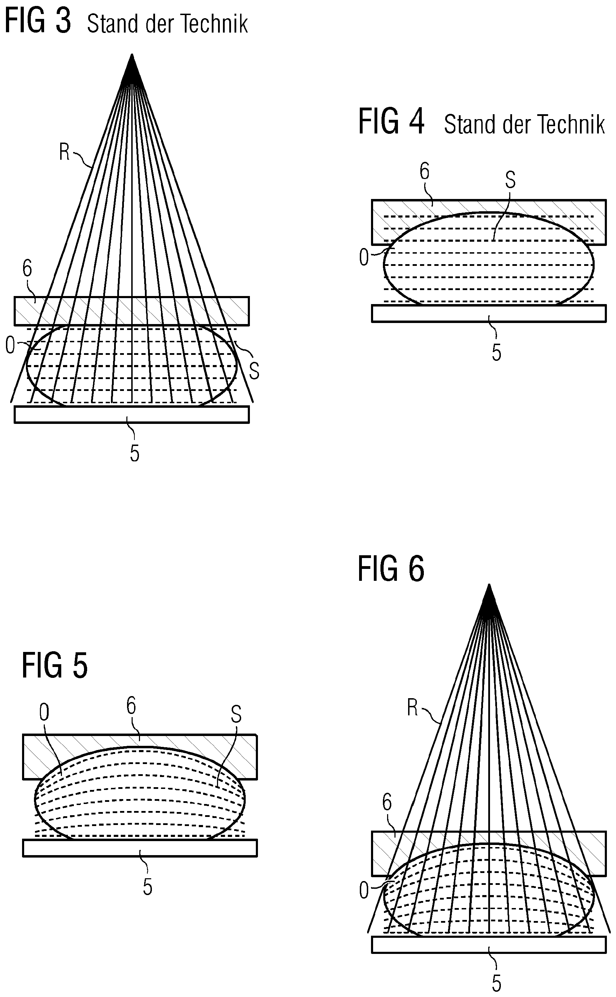

Eine Brust O der Patientin (hier schematisch dargestellt) liegt als Untersuchungsobjekt O für eine Untersuchung oberseitig auf der Detektorfläche 5.1 auf. Über der Brust O und der Detektorfläche 5.1 ist eine Kompressionsplatte 6 angeordnet, die verschiebbar mit der Quelle-Detektor-Anordnung 3 verbunden ist. Für die Untersuchung wird die Brust O komprimiert und zugleich fixiert, indem die Kompressionsplatte 6 auf sie herabgesenkt wird, sodass auf die Brust O zwischen Kompressionsplatte 6 und Detektorfläche 5.1 ein Druck ausgeübt wird. Die der Brust zugewandte Berührungsoberfläche der Kompressionsplatte 6 weist eine konkave Wölbung auf, so dass die Brust dort konvex gewölbt ist, wie z.B. in den Figuren 4, 5 und 6 dargestellt ist.

Der Röntgenstrahler 4 ist dem Detektor 5 gegenüberliegend so angeordnet und ausgebildet, dass der Detektor 5 von ihm emittierte Röntgenstrahlung R erfasst, nachdem zumindest ein Teil der Röntgenstrahlung R die Brust O der Patientin durchdrungen hat. Dabei ist der Röntgenstrahler 4 relativ zum Detektor 5 mittels eines Dreharms 8 in einem Bereich von ± 50° um eine Grundstellung schwenkbar, in der sie senkrecht über der Detektorfläche 5.1 steht. Der aufzunehmende Ausschnitt kann mittels eines Kollimators C vorgegeben bzw. eingeschränkt werden.

Die Steuereinrichtung 9 erhält die Rohdaten RD der Messung und sendet Steuerdaten SD an das Tomosynthesegerät 2 mittels einer Datenschnittstelle. Sie ist mit einem Terminal 20 verbunden, über den ein Benutzer dem Tomosynthesesystem 1 Befehle mitteilen oder Messergebnisse abrufen kann. Die Steuereinrichtung 9 kann in demselben Raum wie das Tomosyn-thesegerät 2 angeordnet sein, es kann sich aber auch in einem angrenzenden Kontrollraum oder in einer noch weiteren räumlichen Entfernung befinden.

Die erfindungsgemäße Vorrichtung 10 zum Erzeugen eines Ergebnisbildes basierend auf einem Tomosynthese-Bilddatensatz der Brust ist in diesem Falle Teil der Steuereinrichtung 9 und umfasst eine Schichteinheit 11, eine Rekonstruktionseinheit 12, eine Ergebniseinheit 13 und eine Datenschnittstelle 14 (s. dazu auch das Verfahren gemäß Figur 2).

Die Schichteinheit 11 definiert gewölbte Schichten S zwischen Kompressionsplatte 6 und Detektor 5, welche in ihrer Form der Wölbung von Kompressionsplatte 6 und/oder Detektor 5 folgen oder einen Übergang zwischen diesen Wölbungen darstellen. Solche Schichten S sind z.B. in den Figuren 5 und 6 dargestellt.

Die Rekonstruktionseinheit 12 dient zur Rekonstruktion von Schichtbildern B, welche auf den gewölbten Schichten S liegen. Die Schichtbilder B sind also im Grunde ebenfalls gewölbt und ergeben einen Schnitt durch die Brust O entlang einer der Schichten S.

Die Ergebniseinheit 13 erzeugt dann ein Ergebnisbild E als Schichtstapel der Schichtbilder B. Beispielsweise arrangiert sie die Schichtbilder B in Form eines Stapels.

Über die Datenschnittstelle 14 wird dann das Ergebnisbild E ausgegeben.

Figur 2 zeigt einen Ablaufplan für einen möglichen Ablauf eines erfindungsgemäßen Verfahrens zum Erzeugen eines Ergebnisbildes E basierend auf einem Tomosynthese-Bilddatensatz T einer Brust O, der z.B. mit dem Mammographiesystem nach Figur 1 aufgenommen worden ist. Links ist angedeutet, wie eine komprimierte Brust aufgenommen und der Tomographiedatensatz T dem Verfahren zur Verfügung gestellt wird.

In Schritt I werden gewölbten Schichten S zwischen Kompressionsplatte 6 und Detektor 5 definiert, welche in ihrer Form der Wölbung von Kompressionsplatte 6 und/oder Detektor 5 folgen oder einen Übergang zwischen diesen Wölbungen darstellen. Der Bereich, in dem bei der Aufnahme die Brust O lag, ist dabei für das Verfahren besonders interessant.

In Schritt II werden dann Schichtbilder B rekonstruiert, welche auf den gewölbten Schichten S liegen. Die Rekonstruktion von Schichtbildern, die Schichten folgen, ist hinlänglich bekannt. Die Besonderheit ist hier, dass an Stelle von ebenen Schichten (s. z.B. Figuren 3 und 4) von gewölbten Schichten S ausgegangen wird.

In Schritt III wird ein Ergebnisbild E als Schichtstapel der Schichtbilder B erstellt. Die Schichten S werden dabei z.B. orthogonal zur Flächennormalen der Berührungsoberfläche des Detektors 5 (also insgesamt horizontal) ausgerichtet. Die Schichtbilder B bilden in Richtung der Flächennormalen (also hier entlang der Vertikalen) aufeinander geschichtet den Schichtstapel.

In Schritt IV wird dieses Ergebnisbild E dann ausgegeben.

Figuren 3 und 4 zeigen ein Beispiel für eine komprimierte Brust O mit einer Rekonstruktion mit ebenen Schichten gemäß dem Stand der Technik.

Figur 3 zeigt dabei eine Brust O, die von zwei ebenen Oberflächen komprimiert wird. Hier sind die ebenen Schichten (gestrichelte Linien) vorteilhaft, da sie den Raum zwischen Kompressionsplatte 6 und Detektor 5 gleichmäßig aufteilen.

Im Gegensatz dazu sind die ebenen Schichten in Figur 6, in der die Brust O mit einer gewölbten Kompressionsplatte 6 komprimiert wird, nachteilhaft, da sie oben, wo sich die Wölbung befindet, nur einen kleinen Teil der Brust O betreffen. Rekonstruierte Schichtbilder B würden dort also nur eine kleine Scheibe der Brust O zeigen.

Figuren 5 und 6 zeigen ein Beispiel für eine gewölbt komprimierte Brust O mit einer Rekonstruktion mit gewölbten Schichten S gemäß dem erfindungsgemäßen Verfahren. Die Wölbung einer ersten Schicht S (oben), welche benachbart zur Kompressionsplatte 6 ist, entspricht dabei im Wesentlichen der Form der Berührungsoberfläche der Kompressionsplatte 6. Die Wölbung einer zweiten Schicht S (unten), welche benachbart zum Detektor 5 ist, entspricht im Wesentlichen der Form der Berührungsoberfläche des Detektors 5. Die Wölbung der dazwischenliegenden Schichten S folgt einem regelmäßigen Übergang zwischen der Wölbung der ersten Schicht S und der zweiten Schicht S. Hier ist die Berührungsoberfläche des Detektors 5 eben und damit die zweite Schicht S ebenfalls eben.

Es wird abschließend noch einmal darauf hingewiesen, dass es sich bei der vorhergehend detailliert beschriebenen Erfindung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen Begriffe wie "Einheit" nicht aus, dass die betreffenden Komponenten aus mehreren zusammenwirkenden Teil-Komponenten bestehen, die ggf. auch räumlich verteilt sein können. Der Begriff "eine Anzahl" ist als "mindestens ein(e)" zu lesen. Unabhängig vom grammatikalischen Geschlecht eines bestimmten Begriffes sind Personen mit männlicher, weiblicher oder anderer Geschlechteridentität mit umfasst.

## Patentansprüche

1. Verfahren zum Erzeugen eines Ergebnisbildes (E) basierend auf einem Tomosynthese-Bilddatensatz (T) einer Brust (O), welche mit einer Kompressionsplatte (6) auf einem Detektor (5) fixiert worden ist, wobei die der Brust (O) zugewandte Berührungsoberfläche von Kompressionsplatte (6) und/oder Detektor (5) eine konkave Wölbung aufweist, das Verfahren umfassend die Schritte:
- Definieren von gewölbten Schichten (S) zwischen Kompressionsplatte (6) und Detektor (5), welche in ihrer Form der Wölbung von Kompressionsplatte (6) und/oder Detektor (5) folgen oder einen Übergang zwischen diesen Wölbungen darstellen,
- Rekonstruieren von Schichtbildern (B), welche auf den gewölbten Schichten (S) liegen
- Erzeugen des Ergebnisbildes (E) als Schichtstapel der Schichtbilder (B),
- Ausgeben des Ergebnisbildes (E).

2. Verfahren nach Anspruch 1, wobei die Wölbung einer ersten Schicht (S), welche benachbart zur Kompressionslatte (6) ist, im Wesentlichen der Form der Berührungsoberfläche der Kompressionsplatte (6) entspricht und die Wölbung einer zweiten Schicht (S), welche benachbart zum Detektor (5) ist, im Wesentlichen der Form der Berührungsoberfläche des Detektors (5) entspricht und die Wölbung der dazwischenliegenden Schichten (S) einem regelmäßigen Übergang zwischen der Wölbung der ersten Schicht (S) und der zweiten Schicht (S) folgt,
bevorzugt wobei die Berührungsoberfläche des Detektors (5) eben ist und die zweite Schicht (S) ebenfalls eben ist und die Wölbung der weiteren Schichten (S) von der zweiten Schicht (S) ausgehend bis zur ersten Schicht (S) stetig zunimmt.

3. Verfahren nach Anspruch 2, wobei die Wölbung der ersten Schicht (S) und/oder der zweiten Schicht (S) basierend auf einer Vermessung der jeweils benachbarten Berührungsoberfläche bestimmt wird oder basierend auf Daten eines Sensors, welcher die Form der Oberfläche der Brust (O) misst.

4. Verfahren nach einem der vorangehenden Ansprüche, wobei für die gewölbten Schichten (S) ein Koordinatensystem (xₙ, yₙ, zₙ) konstruiert wird, bei dem die (xₙ, yₙ) bei gleichem n jeweils in einer Schicht (S) liegen und die zₙ über die Fläche (xₙ, yₙ) die Position der Punkte der gewölbten Schicht (S) vorgeben,
bevorzugt wobei Koordinaten (xₙ, yₙ, zₙ) mittels einer vorgegebene Funktion F berechnet werden, besonders bevorzugt aus einer Vielzahl von Funktionen Fₙ für einzelne Schichten (S), einer von n abhängigen Funktion oder einer von z abhängigen Funktion, wobei für die z insbesondere Koordinaten von Punkten auf ebenen Schichten gewählt werden.

5. Verfahren nach Anspruch 4, wobei die Rekonstruktion von Schichtbildern (B) auf einer Berechnung der Wege von Röntgenstrahlen basiert, die durch die gewölbten Schichten (S) dringen, bevorzugt wobei für eine Projektion der Schichten (S) Koordinatenpaare zwischen einem Koordinatensystem (xₙ, yₙ, zₙ) und einem Koordinatensystem (x, y, z) bestimmt werden, in dem eine Vielzahl von Strahlen mit den Winkeln (α ,φ) zwischen ihren Schnittpunkten mit dem Detektor und der Objektoberfläche in äquidistanten Schritten abgetastet werden.

6. Verfahren nach Anspruch 4 oder 5, wobei die Rekonstruktion die Kegelform eines aufnehmenden Röntgenstrahls berücksichtigt, wobei die Position einer Vielzahl von Punkten (xₙ, yₙ, zₙ) der gewölbten Schichten (S) bestimmt werden, und eine Korrekturrechnung zur Kompensation der Kegelform des Röntgenstrahls für diese Punkte vorgenommen wird.

7. Verfahren nach einem der vorangehenden Ansprüche, wobei als Ergebnisbild (E) ein dreidimensionales Bild der Brust in kartesischen Koordinaten erstellt wird, bevorzugt wobei Bildkoordinaten der Schichtbilder (B) mittels einer linearen Transformation in kartesische Koordinaten überführt werden.

8. Verfahren nach einem der vorangehenden Ansprüche, wobei die Schichten (S) orthogonal zur Flächennormalen der Berührungsoberfläche des Detektors (5) ausgerichtet werden und die Schichtbilder (B) in Richtung dieser Flächennormalen aufeinander geschichtet den Schichtstapel bilden.

9. Vorrichtung (10) zum Erzeugen eines Ergebnisbildes (E) basierend auf einem Tomosynthese-Bilddatensatz (T) einer Brust (O), welche mit einer Kompressionsplatte (6) auf einem Detektor (5) fixiert worden ist, wobei die der Brust (O) zugewandte Berührungsoberfläche von Kompressionsplatte (6) und/oder Detektor (5) eine konkave Wölbung aufweist, die Vorrichtung (10) umfassend:
- eine Schichteinheit (11) ausgelegt zum Definieren von gewölbten Schichten (S) zwischen Kompressionsplatte (6) und Detektor (5), welche in ihrer Form der Wölbung von Kompressionsplatte (6) und/oder Detektor (5) folgen oder einen Übergang zwischen diesen Wölbungen darstellen,
- eine Rekonstruktionseinheit (12), ausgelegt zum Rekonstruieren von Schichtbildern (B), welche auf den gewölbten Schichten (S) liegen
- eine Ergebniseinheit (13), ausgelegt zum Erzeugen des Ergebnisbildes (E) als Schichtstapel der Schichtbilder (B),
- eine Datenschnittstelle (14) ausgelegt zum Ausgeben des Ergebnisbildes (E).

10. Mammographiesystem (1) umfassend eine Vorrichtung (10) nach Anspruch 9 und/oder ausgelegt zum Durchführen eines Verfahrens nach einem der Ansprüche 1 bis 8.

11. Computerprogrammprodukt, umfassend Befehle, die bei der Ausführung des Programms durch einen Computer diesen veranlassen, die Schritte des Verfahrens nach einem der Ansprüche 1 bis 8 auszuführen.

12. Computerlesbares Speichermedium, umfassend Befehle, die bei der Ausführung durch einen Computer diesen veranlassen, die Schritte des Verfahrens nach einem der Ansprüche 1 bis 8 auszuführen.

## Claims

1. Method for generating a result image (E) based on a tomosynthesis image dataset (T) of a breast (0), which has been fixed by a compression plate (6) to a detector (5), wherein the contact surface of the compression plate (6) and/or detector (5) facing the breast (0) has a concave curvature, the method comprising the steps:
- defining curved slices (S) between the compression plate (6) and detector (5), which follow the curvature of the compression plate (6) and/or detector (5) in their shape or represent a transition between these curvatures,
- reconstructing slice images (B) that lie on the curved slices (S)
- generating the result image (E) as a slice stack of the slice images (B),
- outputting the result image (E).

2. Method according to claim 1, wherein the curvature of a first slice (S), which is adjacent to the compression plate (6), substantially corresponds to the shape of the contact surface of the compression plate (6) and the curvature of a second slice (S), which is adjacent to the detector (5), substantially corresponds to the shape of the contact surface of the detector (5) and the curvature of the intermediate slices (S) follows a regular transition between the curvature of the first slice (S) and the second slice (S),
preferably wherein the contact surface of the detector (5) is flat and the second slice (S) is likewise flat and the curvature of the further slices (S) increases steadily from the second slice (S) to the first slice (S).

3. Method according to claim 2, wherein the curvature of the first slice (S) and/or the second slice (S) is determined based on a measurement of the adjacent contact surface in each case or based on data from a sensor which measures the shape of the surface of the breast (0).

4. Method according to one of the preceding claims, wherein a coordinate system (xₙ, yₙ, zₙ) is constructed for the curved slices (S), in which system the (xₙ, yₙ) in each case lie in a slice (S) at the same n and the zₙ above the area (xₙ, yₙ) specify the position of the points of the curved slice (S), preferably wherein coordinates (xₙ, yₙ, zₙ) are calculated by means of a specified function F, particularly preferably from a plurality of functions Fₙ for individual slices (S), a function dependent on n or a function dependent on z, wherein coordinates of points on flat slices are in particular selected for the z.

5. Method according to claim 4, wherein the reconstruction of slice images (B) is based on a calculation of the paths of X-rays penetrating the curved slices (S), preferably wherein, for a projection of the slices (S), coordinate pairs are determined between a coordinate system (xₙ, yₙ, zₙ) and a coordinate system (x, y, z) in which a plurality of rays with the angles (α ,φ) between their points of intersection with the detector and the surface of the object are scanned in equidistant steps.

6. Method according to claim 4 or 5, wherein the reconstruction takes into account the conical shape of a receiving X-ray beam, wherein the position of a plurality of points (xₙ, yₙ, zₙ) of the curved slices (S) are determined and a correction calculation for compensating the conical shape of the X-ray beam is carried out for these points.

7. Method according to one of the preceding claims, wherein a three-dimensional image of the breast in Cartesian coordinates is created as a result image (E), preferably wherein image coordinates of the slice images (B) are converted into Cartesian coordinates by means of linear transformation.

8. Method according to one of the preceding claims, wherein the slices (S) are aligned orthogonally to the surface normal of the contact surface of the detector (5) and the slice images (B) are stacked in the direction of this surface normal to form the slice stack.

9. Apparatus (10) for generating a result image (E) based on a tomosynthesis image dataset (T) of a breast (0), which has been fixed by a compression plate (6) to a detector (5), wherein the contact surface of the compression plate (6) and/or detector (5) facing the breast (0) has a concave curvature, the apparatus (10) comprising:
- a slice unit (11) designed to define curved slices (S) between the compression plate (6) and detector (5), which follow the curvature of the compression plate (6) and/or detector (5) in their shape or represent a transition between these curvatures,
- a reconstruction unit (12) designed to reconstruct slice images (B) lying on the curved slices (S),
- a result unit (13) designed to generate the result image (E) as a slice stack of the slice images (B),
- a data interface (14) designed to output the result image (E) .

10. Mammography system (1) comprising an apparatus (10) according to claim 9 and/or designed to perform a method according to one of claims 1 to 8.

11. Computer program product comprising instructions which, when the program is executed by a computer, cause the computer to execute the steps of the method according to one of claims 1 to 8.

12. A computer-readable storage medium comprising instructions which, when executed by a computer, cause the computer to execute the steps of the method according to one of claims 1 to 8.

## Revendications

1. Procédé de production d'une image (E) de résultat sur la base d'un ensemble (T) de données d'images de tomosynthèse d'un sein (0), qui a été immobilisé sur un détecteur (5) par une plaque (6) de compression, dans lequel la surface de touché, tournée vers le sein (0), de la plaque (6) de compression et/ou du détecteur (5) a une voussure concave, le procédé comprenant les stades :
- définition de couches (S) incurvées entre la plaque (6) de compression et le détecteur (5), qui suivent dans leur forme la voussure de la plaque (6) de compression et/ou du détecteur (5) ou qui représentent une transition entre ces voussures,
- reconstruction d'images (B) de couche, qui se trouvent les couches (S) incurvées,
- production de l'image (E) de résultat comme empilement de couches des images (B) de couche,
- sortie de l'image (E) de résultat.

2. Procédé suivant la revendication 1, dans lequel la voussure d'une première couche (S), qui est voisine de la plaque (6) de compression, correspond sensiblement à la forme de la surface de touché de la plaque (6) de compression et la voussure d'une deuxième couche (S), qui est voisine du détecteur (5), correspond sensiblement à la forme de la surface de touché du détecteur (5) et la voussure des couches (S) intermédiaires suit un passage régulier entre la voussure de la première couche (S) et de la deuxième couche (S)),
dans lequel, de préférence la surface de touché du détecteur (5) est plane et la deuxième couche (S) est également plane, et la voussure des autres couches (S) augmente constamment à partir de la deuxième couche (S) jusqu'à la première couche (S).

3. Procédé suivant la revendication 2, dans lequel on détermine la voussure de la première couche (S) et/ou de la deuxième couche (S) sur la base d'une mesure de la surface de touché voisine respective ou sur la base de données d'un capteur, qui mesure la forme de la surface du sein (O).

4. Procédé suivant l'une des revendications précédentes, dans lequel on construit, pour les couches (S) incurvées, un système (xₙ, yₙ, zₙ) de coordonnées, dans lequel les (xₙ, yₙ) se trouvent pour un même n respectivement dans une couche (S) et les zₙ prescrivent, sur la surface (xₙ, yₙ), la position des points de la couche (S) incurvée,
dans lequel, de préférence on calcule des coordonnées (xₙ, yₙ, zₙ) au moyen d'une fonction F donnée à l'avance, en particulier de préférence à partir d'une pluralité de fonctions Fₙ pour diverses couches (S), d'une fonction qui dépend de n ou d'une fonction qui dépend de z, dans lequel, pour les z, on sélectionne, en particulier des coordonnées de points sur des couches planes.

5. Procédé suivant la revendication 4, dans lequel la reconstruction d'images (B) de couche repose sur un calcul des trajets de rayons X, qui traversent les couches (S) incurvées, dans lequel, pour une projection des couches (S), on détermine des paires de coordonnées entre un système (xₙ, yₙ, zₙ) de coordonnées et un système (x, y, z) de coordonnées, dans lequel on balaye en des points équidistants une pluralité de rayons ayant des angles (α, φ) entre leurs points d'intersection avec le détecteur et la surface objet.

6. Procédé suivant la revendication 4 ou 5, dans lequel la reconstruction tient compte de la forme conique d'un faisceau de rayons X d'enregistrement, dans lequel on détermine la position d'une pluralité de points (xₙ, yₙ, zₙ) des couches (S) incurvées, et on effectue un calcul de correction pour la compensation de la forme conique du faisceau de rayons X pour ces points.

7. Procédé suivant l'une des revendications précédentes, dans lequel on établit, comme image (E) de résultat, une image en trois dimensions du sein dans des coordonnées cartésiennes, dans lequel, de préférence on transforme les coordonnées des images (B) de couche en des coordonnées cartésiennes au moyen d'une transformation linéaire.

8. Procédé suivant l'une des revendications précédentes, dans lequel on oriente les couches (S) orthogonalement à la normale à la surface de touché du détecteur (5) et les images (B) de couche forment l'empilement de couches empilées les unes sur les autres dans la direction de cette normale à la surface.

9. Installation (10) de production d'une image (E) de résultat sur la base d'un ensemble (T) de données d'images de tomosynthèse d'un sein (O), qui a été immobilisé sur un détecteur (5) par une plaque (6) de compression, dans laquelle la surface de touché, tournée vers le sein (0), de la plaque (6) de compression et/ou du détecteur (5), a une courbure concave, l'installation (10) comprenant :
- une unité (11) de couche conçue pour définir des couches (S) incurvées entre la plaque (6) de compression et le détecteur (5), qui suivent dans leur forme la courbure de la plaque (6) de compression et/ou du détecteur (5) ou une transition entre ces courbures,
- une unité (12) de reconstruction conçue pour la reconstruction d'images (B) de couche, qui reposent sur les couches (S) incurvées,
- une unité (13) de résultat conçue pour la production de l'image (E) de résultat comme empilement des images (B) de couche,
- une interface (14) de données conçue pour sortir l'image (E) de résultat.

10. Système (1) de mammographie comprenant une installation (10) suivant la revendication 9 et/ou conçue pour effectuer un procédé suivant l'une des revendications 1 à 8.

11. Produit de programme d'ordinateur, comprenant des instructions qui, lors de l'exécution du programme par un ordinateur, font que celui-ci exécute les stades du procédé suivant l'une des revendications 1 à 8.

12. Support de mémoire exploitable par ordinateur, comprenant des instructions qui, lors de l'exécution par un ordinateur, font que celui-ci exécute les stades du procédé suivant l'une des revendications 1 à 8.
